(19) Europäisches Patentamt
European Patent Office
Office européen des brevets

(11) Veröffentlichungsnummer: **0 243 886**
**A2**

(12) # EUROPÄISCHE PATENTANMELDUNG

(21) Anmeldenummer: **87105973.9**

(22) Anmeldetag: **23.04.87**

(51) Int.Cl.³: **C 07 D 279/16**
A 61 K 31/54
//C07D417/12

(30) Priorität: **28.04.86 DE 3614355**

(43) Veröffentlichungstag der Anmeldung:
**04.11.87 Patentblatt 87/45**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lerch, Ulrich, Dr.**
**Vorderwart 24**
**D-6238 Hofheim am Taunus(DE)**

(72) Erfinder: **Henning, Rainer, Dr.**
**Rotenhofstrasse 31**
**D-6234 Hattersheim am Main(DE)**

(72) Erfinder: **Kaiser, Joachim, Dr.**
**Fichtestrasse 12**
**D-6000 Frankfurt am Main(DE)**

(54) **Neue Benzothiazinon-Derivate, Verfahren zu ihrer Herstellung, sie enthaltende Arzneimittel und deren Verwendung.**

(57) Benzothiazinon-Derivate I

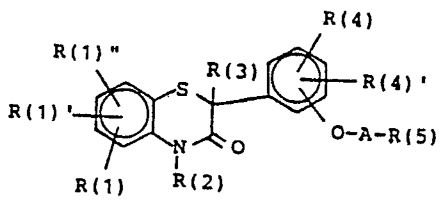

(I)

mit

$R^1$ und $R^4$ gleich H, Alkyl, Alkoxy, Hal, $CF_3$, $NO_2$, OH, Acetamido, Amino;

$R^2$ gleich H, (Cyclo)Alk(en)yl, Cycloalkylalkyl, Phenyl, Phenylalkyl;

A gleich $(CH_2)_mO(CH_2)_n$, $(CH_2)_p$-CH(OH)-$(CH_2)_q$, $(CH_2)_p$-CH($OCH_3$)-$(CH_2)_q$, $(CH_2)_rOCH_2$-CO-, $(CH_2)_s$-CO-;

$R^5$ gleich NR(6)R(7) oder einige cyclische Amine zeigen calciumantagonistische Wirkung.

Man erhält sie unter anderem aus II

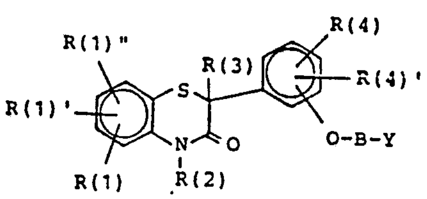

(II),

durch Umsetzung mit III

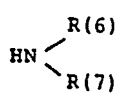

IIIa

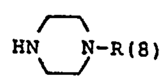

IIIb

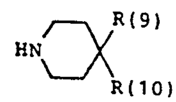

IIIc

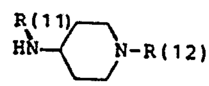

IIId

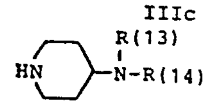

IIIe

EP 0 243 886 A2

Neue Benzothiazinon-Derivate, Verfahren zu ihrer Herstellung, sie enthaltende Arzneimittel und deren Verwendung

Es ist bekannt, daß Verbindungen, die das Einströmen von Calcium-Ionen in Zellen behindern, als Therapeutika zur Behandlung von verschiedenen Krankheiten, insbesondere des Herz-Kreislauf-Systems beim Menschen und anderen Warmblütern eingesetzt werden können.

Benzothiazinon-Derivate mit calciumantagonistischer Wirkung sind in US 4 584 300 beschrieben; die dort aufgeführten Verbindungen sind in der 2-Position des Heterocyclus unsubstituiert.

Weiterhin finden sich Benzothiazinon-Derivate mit calciumantagonistischer Wirkung in der US 4 595 685 Dort werden Verbindungen beschrieben, die am 2-Phenylrest eine basische Ethergruppierung tragen, wobei der basische Stickstoff über eine geradkettige oder verzweigte Alkylkette mit dem Ethersauerstoff verbunden ist.

Wir haben nun überraschend gefunden, daß Verbindungen mit veränderter Seitenkette am 2-Phenylrest überlegene calciumantagonistische Eigenschaften aufweisen.

Gegenstand der Erfindung sind daher neue Benzothiazinon-Derivate der Formel I, die calciumantagonistische Wirkung aufweisen

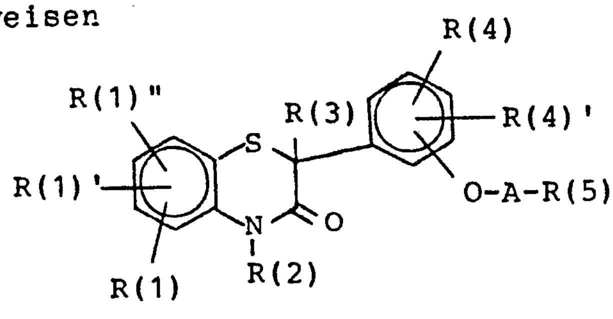

(I)

0243886

R(1), R(1)' und R(1)" gleich oder verschieden und voneinander unabhängig sind und Wasserstoff, $(C_1-C_4)$-Alkyl, $(C_1-C_3)$-Alkoxy, F, Cl, Br, $CF_3$, Nitro, Hydroxy, Acetamido oder Amino,

R(2) Wasserstoff, $(C_1-C_{10})$-Alkyl, geradkettig oder verzweigt, $(C_3-C_{10})$-Alkenyl, geradkettig oder verzweigt, Phenyl-$(C_1-C_4)$-alkyl, wobei der Phenylring unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(3) Wasserstoff, $(C_1-C_{15})$-Alkyl, geradkettig oder verzweigt, $(C_3-C_{15})$-Alkenyl, geradkettig oder verzweigt, $(C_4-C_8)$-Cycloalkyl, $(C_4-C_8)$-Cycloalkyl-$(C_1-C_4)$alkyl, Phenyl oder Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist

R(4) und R(4)' gleich oder verschieden und voneinander unabhängig Wasserstoff, $(C_1-C_4)$-Alkyl, $(C_1-C_3)$-Alkoxy, F, Cl, $CF_3$, Nitro, Hydroxy, Acetamido oder Amino,

A die Gruppe $-(CH_2)_m-O-(CH_2)_n-$, worin m und n jeweils 2 oder 3 ist, oder eine der Gruppen $-(CH_2)_p-CH(OH)-(CH_2)_q-$ und $-(CH_2)_p-CH(OCH_3)-(CH_2)_q$ worin p und q jeweils 1, 2 oder 3 ist, oder die Gruppe $-(CH_2)_r-O-CH_2-CO-$, worin r 2 oder 3 ist, oder die Gruppe $-(CH_2)_s-CO-$, worin s 1, 2, 3 oder 4 ist

R(5) eine der folgenden Gruppen

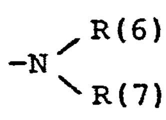
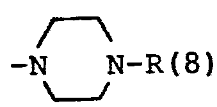
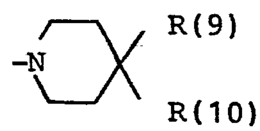

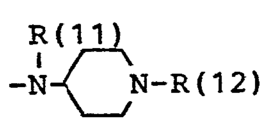
oder
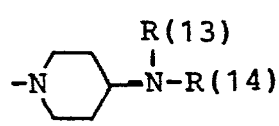

worin

R(6) und R(7) gleich oder verschieden voneinander unabhängig Wasserstoff, $(C_1-C_{10})$-Alkyl, $(C_4-C_8)$-Cycloalkyl, $(C_4-C_8)$-Cycloalkyl-$(C_1-C_4)$-alkyl, Pyridyl-$(C_1-C_4)$-alkyl, Phenyl-$(C_1-C_6)$-alkyl, Benzhydryl oder Benzhydryl-$(C_1-C_4)$-alkyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(8) Wasserstoff, $(C_1-C_{10})$-Alkyl, geradkettig oder verzweigt, $(C_1-C_8)$-Alkanoyl, Pyridyl, Pyrimidinyl, Phenyl, Phenyl-$(C_1-C_4)$-alkyl, Phenyl-$(C_3-C_5)$-alkenyl, Benzhydryl oder Benzylhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(9) Wasserstoff, $(C_1-C_{10})$-Alkyl, Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$ Alkoxy, $(C_1-C_2)$-Alkylendioxy, F, Cl, Br, $CF_3$ oder Hydroxy substituiert ist,

R(10) Wasserstoff, Hydroxy, oder $(C_1-C_4)$-Alkoxy, und

R(11) und R(12) bzw. R(13) und R(14) gleich oder verschieden und voneinander unabhängig Wasserstoff, $(C_1-C_{10})$-Alkyl, geradkettig oder verzweigt, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl, oder Benzoyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

bedeuten,

sowie die Salze der Verbindungen der Formel I mit pharmazeutisch akzeptablen Säuren.

Bevorzugt werden die Verbindungen der Formel I, in welcher

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido,

R(1)" Wasserstoff,

R(2) Wasserstoff, $(C_1-C_6)$-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, $(C_1-C_{12})$-Alkyl, geradkettig oder verzweigt, Allyl, Methallyl, $(C_5-C_7)$Cycloalkyl, $(C_5-C_7)$-Cycloalkyl-$(C_1-C_4)$-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor, Nitro, Hydroxy, Acetamido oder Amino,

R(4)' Wasserstoff,

A die Gruppe $-(CH_2)_m-O-(CH_2)_n-$, worin m und n jeweils 2 oder 3 ist, oder eine der Gruppen $-(CH_2)_p-CH(OH)-(CH_2)_q-$ und $-(CH_2)_p-CH(OCH_3)-(CH_2)_q$ worin p und q jeweils 1, 2 oder 3 ist, oder die Gruppe $-(CH_2)_r-O-CH_2-CO-$, worin r 2 oder 3 sein kann, oder die Gruppe $-(CH_2)_s-CO-$, worin s 1, 2, 3 oder 4 ist

R(5) eine der folgenden Gruppen

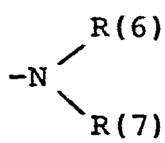
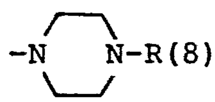
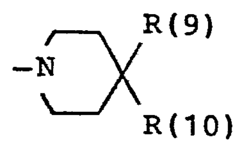

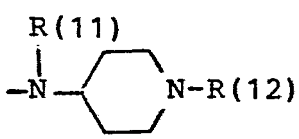 oder 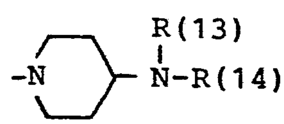

worin

R(6) Wasserstoff, Methyl, Ethyl, Propyl, Isopropyl,

R(7) Wasserstoff, Methyl, Ethyl, Propyl, Isopropyl, Cyclopentylethyl, Cyclohexylethyl, Phenyl-$(C_1-C_4)$-alkyl,

Benzhydryl oder Benzhydryl-($C_1$-$C_4$)-alkyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert sind, Pyridyl-($C_1$-$C_4$)-alkyl,

R(8) Wasserstoff, ($C_1$-$C_6$)-Alkyl, geradkettig oder verzweigt, ($C_1$-$C_6$)-Alkanoyl, Phenyl, wobei der Phenylrest durch einen oder zwei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert sein kann, Phenyl-($C_1$-$C_4$)-alkyl, Phenyl-($C_3$-$C_5$)-alkenyl, Benzhydryl oder Benzhydryl—($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Ethyl, Methoxy, Ethoxy, ($C_1$-$C_2$)-Alkylendioxy, F, Cl, $CF_3$ oder Hydroxy substituiert sind,

R(9) Phenyl, Phenyl-($C_1$-$C_4$)-alkyl, wobei der Phenylrest jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert ist,

R(10) Wasserstoff, Hydroxy oder Methoxy

R(11), R(12), R(13) und R(14) gleich oder verschieden und Wasserstoff, ($C_1$-$C_8$)-Alkyl, ($C_1$-$C_6$)-Alkanoyl, Phenyl-($C_1$-$C_4$)-alkyl, Benzhydryl oder Benzhydryl-($C_1$-$C_4$)-alkyl, wobei die Phenylreste jeweils unsubstituiert oder durch einen, zwei oder drei Reste aus der Gruppe Methyl, Ethyl, Methoxy, Ethoxy, ($C_1$-$C_2$)-Alkylendioxy, F, Cl, $CF_3$ oder Hydroxy substituiert sein können, Phenyl-($C_1$-$C_4$)-alkanoyl oder Benzoyl, wobei die Phenylreste jeweils unsubstituiert oder durch einen, zwei oder drei Reste der Gruppe Methyl, Ethyl, Methoxy, Ethoxy, ($C_1$-$C_2$)-Alkylendioxy, F, Cl, $CF_3$ oder Hydroxy substituiert sind,

bedeuten,

sowie die Salze dieser Verbindungen der Formel I mit pharmazeutisch akzeptablen Säuren.

Besonders bevorzugt werden Verbindungen der Formel I, in welcher

R(1) Wasserstoff, Methyl, Methoxy, Fluor oder Chlor,

R(1)' Wasserstoff oder Methoxy

R(1)" Wasserstoff

R(2) Wasserstoff, Methyl, Ethyl, Propyl, Isopropyl, Butyl, sec. Butyl, Isobutyl, Benzyl, Phenethyl,

R(3) Wasserstoff ($C_1$-$C_{12}$)-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl

R(4) Wasserstoff, Methoxy, Methyl, Chlor, Nitro oder Hydroxy

R(4)' Wasserstoff

A die Gruppe $-(CH_2)_m-O-(CH_2)_n-$, worin m und n jeweils 2 oder 3 ist, oder eine der Gruppen $-(CH_2)_p-CH(OH)-(CH_2)_q-$ und $-(CH_2)_p-CH(OCH_3)-(CH_2)_q$ worin p 1 oder 2 und q 1 ist, oder die Gruppe $-(CH_2)_r-O-CH_2-CO-$, worin r 2 oder 3 ist, oder die Gruppe $-(CH_2)_s-CO-$, worin s 3 oder 4 ist

R(5) eine der folgenden Gruppen

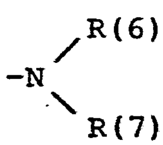 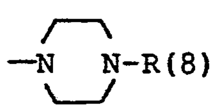 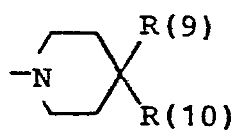

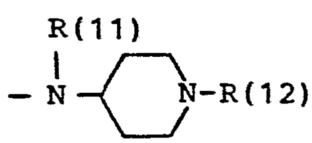 oder 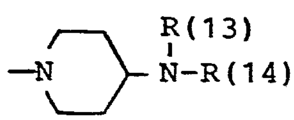

worin

R(6) Wasserstoff oder Methyl

R(7) Phenyl-($C_1$-$C_4$)-alkyl, Benzhydryl oder Benzhydryl-($C_1$-$C_4$) alkyl, wobei der Phenylrest jeweils unsubstituiert oder durch einen, zwei oder drei Reste aus der Gruppe Methyl, Methoxy, Fluor, Chlor, Methylendioxy oder Hydroxy substituiert ist

R(8) $(C_1-C_6)$-Alkyl, geradkettig oder verzweigt, $(C_1-C_6)$-Alkanoyl, Phenyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Methoxy, Ethoxy, Methylendioxy, Fluor, Chlor oder Hydroxy substituiert sind

R(9) Phenyl, wobei der Phenylrest unsubstituiert oder durch einen, zwei oder drei Reste aus der Gruppe Methyl, Methoxy, Fluor, Chlor, Methylendioxy oder Hydroxy substituiert ist

R(10) Wasserstoff, Hydroxy oder Methoxy

R(11), R(12), R(13) und R(14) gleich oder verschieden sind und Wasserstoff, $(C_1-C_6)$-Alkyl, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Methoxy, Methylendioxy, Fluor, Chlor oder Hydroxy substituiert sind,

bedeuten

sowie die Salze dieser Verbindungen der Formel I mit pharmazeutisch akzeptablen Säuren.

Als solche pharmazeutisch akzeptablen Säuren kommen anorganische Säuren wie Salzsäure, Bromwasserstoffsäure, Jodwasserstoffsäure, Schwefelsäure, Phosphorsäure oder Salpetersäure oder organische Säuren wie Weinsäure, Äpfelsäure, Milchsäure, Maleinsäure, Fumarsäure, Malonsäure, Oxalsäure, Gluconsäure, Camphersulfonsäure, Benzolsulfonsäure, Essigsäure, Propionsäure oder p-Toluolsulfonsäure in Betracht.

Die Verbindungen der Formel I weisen asymmetrische C-Atome auf und können daher als Enantiomere oder Diastereomere auftreten. Die Erfindung umfaßt sowohl die reinen Isomeren als auch deren Gemische. Diese Gemische von Diastereomeren können nach gebräuchlichen Methoden, zum Beispiel selektive Kristallisation aus geeigneten Lösungsmitteln oder Chromatographie

an Kieselgel oder Aluminiumoxid in die Komponenten aufgetrennt werden. Racemate können nach üblichen Methoden in die einzelnen Enantiomeren aufgetrennt werden, so zum Beispiel durch Salzbildung mit optisch aktiven Säuren wie Camphersulfonsäure oder Dibenzoylweinsäure und selektive Kristallisation, oder durch Derivatisierung mit geeigneten optisch aktiven Reagenzien, Trennung der diastereomeren Derivate und Rückspaltung.

Die Erfindung betrifft weiterhin Verfahren zur Herstellung von Verbindungen der Formel I, dadurch gekennzeichnet, daß man

a) eine Verbindung der Formel II,

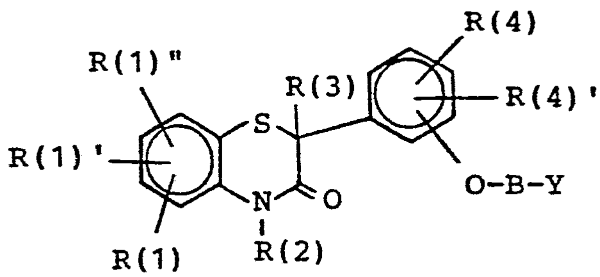

(II)

in welcher R(1), R(1)', R(1)", R(2), R(3), R(4) und R(4)' die gleiche Bedeutung wie in Formel I haben und in welcher B die Reste $-(CH_2)_m-O-(CH_2)_n$ oder $-(CH_2)_p-CH(OH)-(CH_2)_q$ bedeutet, wobei m, n, p und q die gleiche Bedeutung wie in Formel I haben, und in welcher Y eine Abgangsgruppe, die nucleophil verdrängt werden kann, insbesondere ein Chlor-, Brom- oder Jodatom, einen Sulfonsäurerest, vorzugsweise einen Methansulfonylrest, einen Benzolsulfonylrest, einen Toluolsulfonylrest oder einen Trifluormethansulfonylrest bedeutet, mit einer der Verbindungen der Formeln IIIa, IIIb, IIIc, IIId oder IIIe

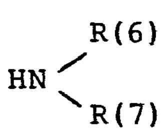

IIIa

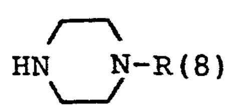

IIIb

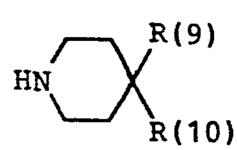

IIIc

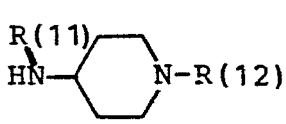

IIId

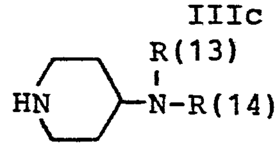

IIIe

in welchen R(6), R(7), R(8), R(9), R(10), R(11), R(12), R(13) und R(14) gleiche Bedeutung wie in Formel I haben, unter Bedingungen einer nucleophilen Substitution, vorzugsweise in einem polaren organischen Lösungsmittel wie einem Alkohol, vorzugsweise Methanol, Ethanol, Propanol oder Isopropanol oder einem niederen Keton, vorzugsweise Aceton oder Methylethylketon oder Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumcarbonat, Natriumcarbonat, Triethylamin, N-Ethylmorpholin oder Pyridin, bei einer Temperatur zwischen 0 und 160°C, vorzugsweise zwischen 20 und 120°C, umsetzt, oder daß man

b) eine Verbindung der Formel IV,

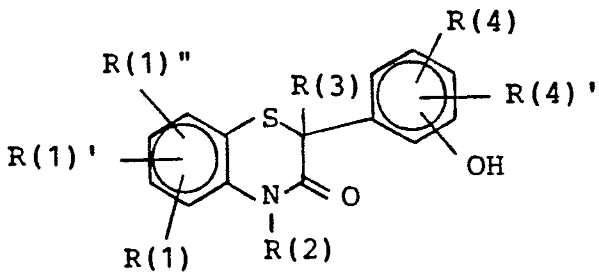

IV

in welcher R(1), R(1)', R(1)", R(2), R(3), R(4) und R(4)' die gleiche Bedeutung wie in Formel I haben, mit einer Verbindung der Formel V,

Z - A - R(5) V

in welcher Z gleich wie Y in Formel II definiert ist und in welcher R(5), und A die gleiche Bedeutung wie in Formel I haben, entweder in einem polaren aprotischen Lösungsmittel wie Dimethylformamid, Dimethylsulfoxid, Tetrahydrofuran, Sulfolan oder N-Methylpyrrolidon, in Gegenwart einer starken Base wie Natriumhydrid, Kaliumhydrid, Natriumamid, Lithiumdiisopropylamid, Butyllithium oder Lithiumhexamethyldisilazid, bei einer Temperatur zwischen -40 und +60°C, vorzugsweise zwischen -10 und -30°C, oder in einem protischen oder aprotischen polaren organischen Lösungsmittel wie einem niederen Alkohol, beispielsweise Methanol, Ethanol, Isopropanol oder einem niederen Keton, vorzugsweise Aceton oder Methylethylketon oder in Dimethylformamid, in Gegenwart einer schwachen bis mittelstarken Base wie einem Alkali- oder Erdalkalimetallhydroxid oder -carbonat oder einem Amin wie beispielsweise Triethylamin, N-Ethylmorpholin, N-Methyldiisopropylamin oder Pyridin, bei einer Temperatur zwischen 0 und 160°C, vorzugsweise zwischen 20 und 120°C, umsetzt, oder daß man

c) eine Verbindung der Formel VI

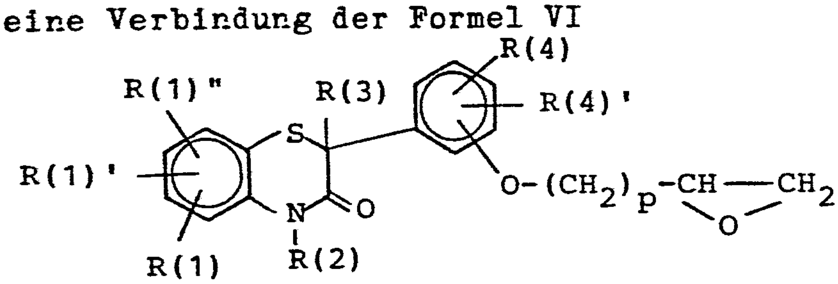

in welcher R(1), R(1)', R(1)" , R(2), R(3), R(4), R(4)' und p die gleiche Bedeutung wie in Formel I haben, mit Aminen der Formel IIIa - IIIe ohne Lösungsmittel oder in Gegenwart eines vorzugsweise polaren Lösungsmitttels wie Methanol, Isopropanol, Aceton, THF oder Dimethylformamid umsetzt, wobei Verbindungen der Formel I entstehen, worin

A = $(CH_2)_p$-CH(OH)-$CH_2$ bedeutet oder daß man

d) eine Verbindung der Formel VII

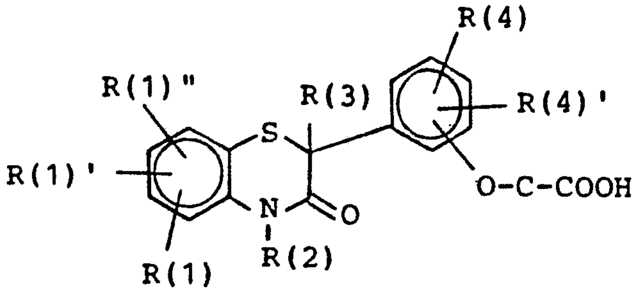

VII

in welcher R(1), R(1)', R(1)", R(2), R(3), R(4), R(4)' die gleiche Bedeutung wie in Formel I haben und in welcher C die Reste $-(CH_2)_r-O-CH_2-CO-$ oder $-(CH_2)_s-CO-$, bedeutet wobei r und s die gleiche Bedeutung wie in Formel I haben,
mit einer der Verbindungen der Formeln IIIa, IIIb, IIIc, IIId oder IIIe unter aus der Literatur bekannten Amidbildungsbedingungen umsetzt, wobei Verbindungen der Formel I entstehen, in denen A $-(CH_2)_r-O-CH_2CO-$ oder $-(CH_2)_s-CO-$ bedeutet.

Verbindungen der Formel II erhält man aus Verbindungen der Formel IV und Verbindungen der Formel VIII

$$Z - B - Y \qquad VIII,$$

worin B und Y die gleiche Bedeutung wie in Formel II haben und Z eine Abgangsgruppe, die nucleophil verdrängt werden kann, wobei Z wie Y definiert ist und gleich Y oder davon verschieden sein kann, unter den unter b) beschriebenen Bedingungen.

Verbindungen der Formel VI erhält man aus Verbindungen der Formel IV z.B. mit Epichlorhydrin und Basen (für p= 1) nach bekannten Methoden oder durch Alkylierung von Verbindungen der Formel IV mit Verbindungen der Formel IX

$$Y - (CH_2)_p-CH=CH_2 \qquad IX$$

worin p die gleiche Bedeutung wie in Formel I hat und

Y die gleiche Bedeutung wie in Formel II hat, wobei Verbindungen der Formel X

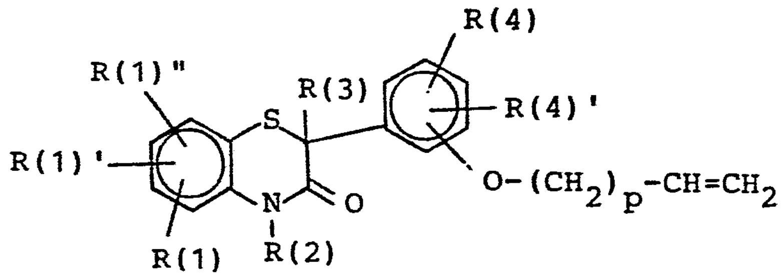

X

gebildet werden, worin R(1), R(1)', R(1)", R(2), R(3), R(4), R(4)' und p die gleiche Bedeutung wie in Formel I haben. Anschließende Epoxidation der Verbindungen X nach bekannten Verfahren, beispielsweise mit m-Chlorperbenzoesäure in Methylenchlorid ergibt Verbindungen der Formel VI.

Verbindungen der Formel VII erhält man aus Verbindungen der Formel IV und Verbindungen der Formel XI

Y - C - COOR(15)          XI

worin Y die gleiche Bedeutung wie in Formel II und C die gleiche Bedeutung wie in Formel VII aufweist und R(15) Wasserstoff, eine ($C_1$-$C_5$)-Alkylgruppe, geradkettig oder verzweigt oder eine gegebenenfalls durch Methoxy oder Nitro substituierte Benzylgruppe darstellt, unter den unter Verfahrensvariante b) beschriebenen Bedingungen und gegebenenfalls anschließende Abspaltung des Ester-Restes R(15) unter literaturbekannten Bedingungen, beispielsweise katalytische Hydrierung oder alkalische Verseifung.

Verbindungen der Formel IV sind aus der EP-A-146 893 bekannt.

Alkyl, Alkylen, Alkanoyl, Alkoxy bedeuten, wenn nicht ausdrücklich anders erwähnt, immer gerade oder verzweigte Ketten.

Die erfindungsgemäßen Verbindungen der Formel I weisen blutdrucksenkende, insbesondere calciumantagonistische Wirkungen auf und können daher zur Behandlung aller Krankheitszustände, die auf einer Störung in dem Calciumhaushalt eines Warmblüters beruhen, verwendet werden.

Ihre calciumantagonistische Wirksamkeit kann an dem biochemischen Testmodell der Verdrängung von tritiummarkiertem Nitrendipin gezeigt werden.

Hierbei werden Membranpräparationen, die isolierte Calciumkanäle enthalten, mit der markierten Substanz beladen. Nach Inkubation mit der Testsubstanz wird die freigesetzte Radioaktivität in der überstehenden Lösung bestimmt. In diesem Modell weisen die erfindungsgemäßen Verbindungen der Formel I $IC_{50}$-Werte von $10^{-6}$ molar bis $10^{-10}$ molar auf. In weiteren Testmodellen, mit denen calciumantagonistische Wirkung nachgewiesen werden kann, z. B. an der Coronardurchströmung am isolierten Meerschweinchenherzen oder am Aktionspotential des isolierten Meerschweinchenpapillarmuskels sind die Verbindungen der Formel I ebenfalls stark wirksam.

Die erfindungsgemäßen Verbindungen der Formel I und ihre pharmakologisch verträglichen Salze vermindern das Einströmen von Calcium-Ionen in Zellen und eignen sich daher zu Behandlung des Herz-Kreislaufsystems bei entsprechenden Beschwerden z. B. bei verschiedenen Formen der Angina pectoris, Tachycardie, Herzrhythmusstörungen und Bluthochdruck. Sie sind innerhalb eines breiten Dosisbereichs wirksam. Die Höhe der verabreichten Dosis ist abhängig von der Art der gewünschten Behandlung, von der Verabreichungsweise, vom Zustand, vom Typ und von der Größe des behandelten Säugers. Bei oraler Dosierung werden befriedigende Ergebnisse mit Dosen von ab 0,01 mg, vorzugsweise

ab 0,1 mg und bis zu 100 mg, vorzugsweise bis zu 20 mg einer Verbindung der Formel I pro kg Körpergewicht erreicht. Beim Menschen variiert die tägliche Dosis zwischen 10 und 800 mg, vorzugsweise 20 bis 500 mg, wobei Einzeldosen von 5 bis 200 mg, vorzugsweise ein bis dreimal täglich, gegeben werden können.

Für intravenöse und intramuskuläre Anwendung beträgt die Dosis 1 bis 300 mg, vorzugsweise 5 bis 150 mg täglich.

Die pharmakologisch verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z. B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Requlierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden z. B. mittels konventioneller Misch-Granulier- und Dragierverfahren, hergestellt und enthalten 0,1 % bis etwa 75 %, bevorzugt etwa 1 % bis etwa 50 % des Wirkstoffs.

Die im Anschluß folgenden Beispiele sollen die Erfindung erläutern, ohne sie auf diese Beispiele zu begrenzen.

Beispiel 1

3,4-Dihydro-2-isopropyl-4-methyl-2-[2-(5-bromo-3-oxa-pentyloxy)-phenyl]-2H-1,4-benzothiazin-3-on

9,4 g (30 mmol) 3,4-Dihydro-2-isopropyl-4-methyl-2-(2-hydroxyphenyl)-2H-1,4-benzothiazin-3-on werden mit 22 g (94 mmol) 1,5-Dibrom-3-oxapentan und 12,9 g (90 mmol) Kaliumcarbonat in 75 ml Butanon-2 8 Stunden am Rückfluß gekocht. Danach wird abgesaugt und eingeengt, zuletzt im Hochvakuum. Man erhält 12,4 g fast farbloses Öl.

$^1$H-NMR ($CDCl_3$): δ= 7,5-6,5 (m, 8H), 4,0-3,3 (m, 8H), 3,4 (s, 3H), 2,9 (Septett, 1H); 1,15 + 0,95 (2d, 6H) ppm.

Beispiel 2

3,4-Dihydro-2-isopropyl-4-methyl-2-[2-[5-[4-[2-(3,4,5-trimethoxyphenyl)-ethyl]-piperazinyl]-3-oxa-pentoxy]-phenyl]-2H-1,4-benzothiazin-3-on-Dihydrochlorid

4,1 g (88 mmol) der Verbindung aus Beispiel 1 werden mit 1,2 g Kaliumcarbonat und 8 g 2-(3,4,5-Trimethoxyphenyl)-ethyl)-piperazin in 100 ml Isopropanol 8 Stunden zum Sieden erhitzt. Nach Abkühlen wird abgesaugt und eingeengt. Chromatographie an 300 g Kieselgel ergibt 4,7 g farbloses Öl.

$^1$H-NMR ($CDCl_3$): δ= 7,5-6,5 (m, 8H), 6,38 (s, 2H), 4,1-3,5 (m, 6H), 3,8 (s, 9H), 3,4 (s, 3H); 3,0-2,4 (m, 16H); 1,13 + 0,95 (2d, 6H);

Die freie Base wird in Methanol gelöst und mit etherischer Salzsäure versetzt. Nach Einengen wird aus Isopropanol/Ether umkristallisiert. Man erhält 3,1 g Dihydrochlorid vom Schmp. 191 - 192°C.

| | | | | | |
|---|---|---|---|---|---|
| Ber. | $C_{37}H_{49}N_3O_6S \cdot 2HCl \cdot H_2O$ | C 58.9 | H 7.08 | N 5.6 | Cl 9.4 |
| Gef. | | C 59.0 | H 7.2 | N 5.5 | Cl 10.1 |

Beispiel 3

3,4-Dihydro-2-isopropyl-4-methyl-2-[2-[5-[4-[4,4-[bis-(4-fluorphenyl) ]-butyl]-piperazinyl]-3-oxa-pentyloxy - phenyl]-2H-1,4-benzothiazin-3-on-Dihydrochlorid

4,1 g (88 mmol) der Verbindung aus Beispiel 1 wurden mit 1,2 g Kaliumcarbonat und 5,6 g 4,4-Bis-(4-fluorphenyl)-butyl-piperazin in 100 ml Isopropanol für 8 Stunden am Rückfluß erhitzt. Aufarbeitung wie in Beispiel 2 ergibt 2,8 g farbloses Öl.

$^1$H-NMR ($CDCl_3$): δ= 7,5-6,4 (m, 16H); 4,1-3,6 (m, 7H); 3,4 (s, 3H); 3,0-1,8 (m, 17H); 1,08 + 0,95 (2d, 6H) ppm

Das Di-Hydrochlorid erhält man durch Versetzen der methanolischen Lösung der freien Base mit etherischer HCl und Einengen. Schmp. 110°C, Ausbeute 3,6 g

| | | | | |
|---|---|---|---|---|
| Ber. | $C_{42}H_{49}N_3O_3F_2S \cdot 2HCl \cdot CH_3OH$ | C 62.3 | H 6.7 | N 5.1 |
| Gef. | | C 61.6 | H 6.6 | N 4.8 |

Beispiel 4

3,4-Dihydro-2-isopropyl-4-methyl-2-[2-[5-[N-methyl-N-[2-(3,4-dimethoxyphenyl)-ethyl]-3-oxa-pentyloxy]-phenyl] - 2H-benzothiazin-3-on-Hydrochlorid

4,1 g (8,8 mmol) der Verbindung aus Beispiel 1 werden mit

1,2 g Kaliumcarbonat und 3,4 g N-Methyl-homoveratrylamin nach dem in Beispiel 2 beschriebenen Verfahren umgesetzt. Man erhält 2,2 g der freien Base als Öl.

$^1$H-NMR ($CDCl_3$): δ= 7,5-6,5 (m, 11H); 4,05-3,6 (m, 6H); 3,88 + 3,80 (2s, 6H); 3,4 (s, 3H); 3,1-2,7 (m, 6H); 2,6 (s, 3H); 1,15 + 0,9 (2d, 6H) ppm.

Das Hydrochlorid erhält man als viskoses Harz durch Versetzen mit etherischer HCl und Einengen.

Beispiel 5

3,4-Dihydro-2-isopropyl-4-methyl-2-[2-(oxiranyl-methoxy)-phenyl]-2H-1,4-benzothiazin-3-on

4,7 g (15,0 mmol) 3,4-Dihydro-2-isopropyl-4-methyl-2-(2-hydroxy-phenyl)-2H-1,4-benzothiazin-3-on werden mit 0,6 g NaOH, 2 ml $H_2O$ und 50 ml Epichlorhydrin 24 Stunden bei 85°C gerührt. Nach Verdünnen mit Wasser wird mit Methylenchlorid extrahiert, mit Natriumsulfat getrocknet und eingeengt. Man erhält 5,3 g der Titelverbindung als farbloses Öl.

$^1$H-NMR ($CDCl_3$): δ= 7,6-6,4 (m, 8H); 4,2-3,8 (m, 3H); 3,4 (s, 3H); 3,4-2,5 (m, 4H); 1,4-0,7 (m, 6H) ppm

Beispiel 6

3,4-Dihydro-2-isopropyl-4-methyl-2-[2-[2-hydroxy-3-[4-[4-[-2-(3,4,5-trimethoxyphenyl)ethyl]-piperazinyl]-propoxy]-phenyl]-2H-1,4-benzothiazin-3-on-Dihydrochlorid

3 g der Verbindung aus Beispiel 5 werden mit 6,8 g (24 mmol) 2-(3,4,5-Trimethoxyphenyl)ethyl-piperazin in 50 ml

Methanol 24 Stunden am Rückfluß gerührt. Nach Säulenchromatographie an 300 g Kieselgel erhält man 18 g freie Base als Öl.

$^1$H-NMR ($CDCl_3$) δ= 7,5-6,5 (m, 8H); 6,45 (s, 2H); 4,1-3,4 (m, 5H); 3,8 (s, 9H); 3,45 (s, 3H); 3,1-2,4 (m, 13H); 1,25 + 0,9 (2d, 6H) ppm

Aus methanolischer Lösung wird mit etherischer Salzsäure das Hydrochlorid gefällt und aus Ethanol umkristallisiert Schmp. 236 - 237°C (Zers.)

Beispiel 7

3,4-Dihydro-2-isopropyl-4-methyl-2-[2-[2-hydroxy-3-[4-[4,4-bis-(4-fluorphenyl)]-butyl]-piperazinyl]-propoxy]-phenyl -2H-1,4-benzothiazin-3-on-Dihydrochlorid

4,4 g (12 mmol) der Verbindung aus Beispiel 5 werden mit 7,9 g 4,4-Bis-(4-fluorphenyl)-butyl-piperazin nach dem in Beispiel 6 beschriebenen Verfahren umgesetzt. Man erhält 2,7 g der freien Base als Öl.

$^1$H-NMR ($CDCl_3$): δ= 7,5-6,5 (m, 16H); 4,3-3,6 (m, 6H); 3,4 (s, 3H); 2,8-2,0 (m, 14H); 1,5-0,7 (m, 6H) ppm.

Dihydrochlorid: farblose Kristalle, Schmp. 85°C (Zers.)

Beispiel 8

3,4-Dihydro-2-isopropyl-4-methyl-2-[2-[2-hydroxy-3-[4-[4-[3-(3,4,5-trimethoxyphenyl)-propyl]-piperazinyl]-propoxy]-phenyl]-2H-1,4-benzothiazin-3-on Dihydrochlorid

5,5 g (15 mmol) der Verbindung aus Beispiel 5 werden mit 6,6 g (22 mmol) 3-(3,4,5-Trimethoxyphenyl)-propyl-piperazin nach dem in Beispiel 5 beschriebenen Verfahren umgesetzt.

Freie Base: farbloses Öl, Ausbeute 3,9 g

$^1$H-NMR ($CDCl_3$): δ= 7,5-6,5 (m, 8H); 6,38 (s, 2H); 4,2-3,6 (m, 5H); 3,75 (s, 9H); 3,4 (s, 3H); 2,8-2,4 (m, 13H); 2,2-1,7 (m, 2H); 1,15 + 0,8 (2d, 6H) ppm

Dihydrochlorid: Farblose Kristalle, Schmp. 105°C

Beispiel 9

7-Chlor-3,4-dihydro-2-isopropyl-4-methyl-2-[2-(oxiranylmethoxy)-phenyl]-2H-benzothiazin-3-on

3 g 7-Chlor-3,4-dihydro-2-isopropyl-4-methyl-2-(2-hydroxyphenyl)-2H-1,4-benzothiazin-3-on werden in eine Suspension von 0,6 g NaH (50 % in Öl) in 40 ml Dimethylformamid gegeben. Nach 15 min werden 2 ml Epichlorhydrin zugefügt und 20 Stunden bei 20°C gerührt. Nach Verdünnen mit Essigester wird 3x mit Wasser und 1x mit gesättigter Kochsalzlösung gewaschen, mit Natriumsulfat getrocknet und eingeengt. Durch Chromatographie an Kieselgel werden die Diastereomeren getrennt (Laufmittel Essigester/Cyclohexan 1:4).

Isomer 1 Ausbeute 1,49 g
Rf: 0,4 (Essigester/Cyclohexan 1:1)
Schmp. 123-125°C (aus Ethanol/Hexan)

$^1$H-NMR($CDCl_3$): δ= 7,6-6,5 (m, 7H); 4,3-3,5 (m, 3H); 3,45 (s, 3H); 3,0-2,4 (m, 3H); 1,1 + 0,95 (2d, 6H) ppm

Isomer 2 Ausbeute 1,35 g
Rf: 0,35 (Essigester/Cyclohexan 1:1)
Schmp. 107-108°C (aus Ethanol/Hexan)

$^1$H-NMR($CDCl_3$): δ= 7,6-6,6 (m, 7H); 4,3-3,6 (m, 2H); 3,5-3,2 (m, 1H); 3,4 (s, 3H); 3,0-2,5 (m, 2H); 1,1 + 0,97 (2d, 6H) ppm

Beispiel 10

7-Chlor-3,4-dihydro-2-isopropyl-4-methyl-2-[2-[2-hydroxy-3-[4-[2-(3,4,5-trimethoxyphenyl)-ethyl]-piperazinyl]-propoxy]- phenyl]-2H-1,4-benzothiazin-3-on-Dihydrochlorid (Isomer 1)

1,2 g Isomer 1 aus Beispiel 9 werden mit 1,5 g 2-(3,4,5-Trimethoxyphenyl)-ethyl-piperazin-Dihydrochlorid und 1,5 g Kaliumcarbonat in 30 ml Isopropanol 8 Stunden am Rückfluß gekocht. Nach Abkühlen wird filtriert und eingeengt. Chromatographie an Kieselgel mit Methylenchlorid/Methanol (20:1) ergibt 1,8 g farbloses Öl.
$^1$H-NMR ($CDCl_3$): δ= 7,5-6,65 (m, 7H); 6,44 (s, 2H); 4,28 (s, 1H); 4,0-3,9 (m, 1H); 3,86 + 3,80 (2s, 9H); 3,8-3,65 (m, 2H); 3,42 (s, 3H); 3,1 (m, 1H); 2,85-2,45 (m, 14H); 1,25 + 0,78 (2d, 6H) ppm

Die freie Base wird in Aceton gelöst, mit 3 ml ethanol. HCl versetzt, eingeengt, noch zweimal mit Aceton eingeengt. Nach Stehenlassen in Aceton werden die Kristalle abgesaugt. Schmp. 215-219°C, Ausbeute 1,54 g

| | | | | |
|---|---|---|---|---|
| Ber. | $C_{36}H_{46}ClH_3O_6S$-2HCl.1/2 $H_2O$ | C 56,4 | H 6,6 | N 5,5 |
| Gef. | | C 56,5 | H 6,5 | N 5,0 |

Beispiel 11

7-Chlor-3,4-dihydro-2-isopropyl-4-methyl-2-[2-[2-hydroxy-3-[4-[2-(3,4,5-trimethoxyphenyl)-ethyl]-piperazinyl]-propoxy]-phenyl-2H-1,4-benzothiazin-3-on-Dihydrochlorid (Isomer 2)

0,9 g des Isomers 2 aus Beispiel 9 werden mit 1,3 g 2-(3,4,5-Trimethoxyphenyl)-ethyl-piperazin-Dihydrochlorid und 1,3 g Kaliumcarbonat in 25 ml Isopropanol nach dem in

Beispiel 10 angegebenen Verfahren umgesetzt. Man erhält 1,25 g freie Base als Öl.
$^1$H-NMR ($CDCl_3$): δ= 7,6-6,5 (m, 7H); 6,45 (s, 2H); 4,3-3,5 (m, 4H); 3,85 (s, 9H); 3,45 (s, 3H); 3,2-2,4 (m, 15H); 1,2 + 0,8 (2d, 6H) ppm
Dihydrochlorid: Amorphes Pulver

Beispiel 12

7-Chlor-3,4-dihydro-2-isopropyl-4-methyl-2-[2-[2-methoxy-3-[4-[2-(3,4,5-trimethoxyphenyl)-ethyl]-piperazinyl]-propoxy]-phenyl]-2H-1,4-benzothiazin-3-on-Dihydrochlorid (Isomer 1)

0,75 g der Verbindung aus Beispiel 10 werden zu einer Suspension von 0,5 g Natriumhydrid (50 % in Öl) in 15 ml DMF gegeben. Nach 10 min werden 70 µl Methyljodid zugefügt und 3 Stunden gerührt. Nach Verdünnen mit Essigester wird 4x mit Wasser gewaschen, mit Natriumsulfat getrocknet und eingeengt. Man erhält 0,38 g Öl.
$^1$H-NMR ($CDCl_3$): δ= 7,6-6,5 (m, 7H); 6,4 (s, 2H); 4,3-3,5 (m, 4H); 3,8 (s, 9H); 3,55 (s, 3H); 3,45 (s, 3H); 3,2-2,4 (m, 15H); 1,15 + 0,9 (2d, 6H) ppm
Dihydrochlorid: Farblose Kristalle, Schmp. 237-240°C

Beispiel 13

7-Chlor-3,4-dihydro-2-isopropyl-4-methyl-2-[2-[2-methoxy-3-[4-[2-(3,4,5-trimethoxyphenyl)-ethyl]-piperazinyl]-propoxy]-phenyl]-2H-1,4-benzothiazin-3-on-Dihydrochlorid (Isomer 2)

0,7 g der Verbindung aus Beispiel 11 werden nach der in Beispiel 12 angegebenen Vorschrift mit 0,5 g Natriumhydrid (50 % in Öl) und 70 µl Methyljodid umgesetzt. Man erhält 0,6 g der Titelverbindung als farbloses amorphes Pulver.

Beispiel 14

4-[(4-Methyl-2-isopropyl-3,4-dihydro-2H-1,4-benzothiazin-3-on-2-yl)-2-phenoxy]-buttersäure-4-[2-(3,4,5-trimethoxyphenyl)-ethyl]-piperazid-Hydrochlorid

a) 15,7 g 3,4-Dihydro-2-isopropyl-4-methyl-2-(2-hydroxyphenyl)-2H-1,4-benzothiazin-3-on werden mit 6,9 g Kaliumcarbonat und 8 ml 4-Brombuttersäureethylester in 150 ml Butanon-2 8 Stunden am Rückfluß gerührt. Nach Absaugen wird eingeengt. Man erhält 19,8 g 4-[(4-Methyl-2-isopropyl-3,4-dihydro-2H-1,4-benzothiazin-3-on-2-yl)-2-phenoxy]-buttersäureethylester.
$^1$H-NMR ($CDCl_3$) δ= 7,5-6,4 (m, 8H); 4,1 (q, 2H); 3,8 (t, 2H); 3,4 (s, 3H); 3,2-2,0 (m, 5H); 1,4-0,7 (2d + t, 9H) ppm

b) 21,4 g des Ester aus a) werden in 250 ml Ethanol mit 3,35 g KOH 3 Stunden am Rückfluß gekocht. Nach Einengen wird in wenig Wasser aufgenommen und 2x mit Essigester extrahiert. Die wäßrige Phase wird mit HCl angesäuert und 3x mit Essigester extrahiert, die Essigesterphasen werden mit Natriumsulfat getrocknet und eingeengt. Man erhält 118 g 4-[(4-Methyl-2-isopropyl-3,4-dihydro-2H-1,4-benzothiazin-3-on-2-yl)-2-phenoxy]-buttersäure vom Schmp. 131-133°C (aus Toluol umkristallisiert).

| | | | | |
|---|---|---|---|---|
| Ber. | $C_{22}H_{25}NO_4S$ | C 66,1 | H 6.3 | N 3.5 |
| Gef. | | C 65,7 | H 6,4 | N 3,5 |

c) 6 g (15 mmol) der Carbonsäure aus b) werden in 20 ml DMF mit 2 ml einer 30 %igen Lösung von 1-Hydroxybenztriazol in DMF versetzt. Bei Zimmertemperatur gibt man 4,2 g (15 mmol) 2-(3,4,5-Trimethoxyphenyl)ethyl-piperazin und 3,7 g (18 mmol) Dicyclohexylcarbodiimid zu. Nach 14 Stunden bei Zimmertemperatur wird abgesaugt, die Lösung

mit 200 ml Eiswasser versetzt und mit Essigester extrahiert (3x). Die vereinigten organischen Phasen werden je 1x mit gesättigter Natriumhydrogencarbonat-Lösung und Wasser gewaschen, mit Natriumsulfat getrocknet und eingeengt. Chromatographie an 250 g Kieselgel mit Methylenchlorid/Methanol (9:1) ergibt die freie Base.

$^1$H-NMR ($CDCl_3$): $\delta$= 7,5-6,5 (m, 8H); 6,4 (s, 2H); 4,2-3,5 (m, 4H); 3,8 (s, 9H); 3,4 (s, 3H); 3,1-2,0 (m, 15H); 1,15 + 0,95 (2d, 6H) ppm
Hydrochlorid: Ausbeute 5,3 g, Schmp. 138-140°C

| | | | | |
|---|---|---|---|---|
| Ber. | $C_{37}H_{47}N_3O_6S \cdot HCl$ | C 62,6 | H 6,9 | N 6,0 |
| Gef. | | C 62,3 | H 7,0 | N 6,0 |

PATENTANSPRÜCHE:

1. Verbindung der Formel I

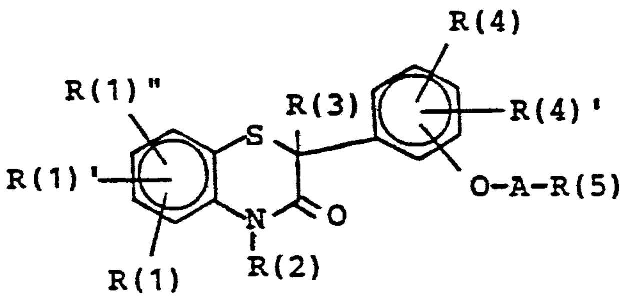

(I),

worin bedeuten

R(1), R(1)' und R(1)", gleich oder verschieden und voneinander unabhängig, Wasserstoff, $(C_1-C_4)$-Alkyl, $(C_1-C_3)$-Alkoxy, F, Cl, Br, $CF_3$, Nitro, Hydroxy, Acetamido oder Amino,

R(2) Wasserstoff, $(C_1-C_{10})$-Alkyl, geradkettig oder verzweigt, $(C_3-C_{10})$-Alkenyl, geradkettig oder verzweigt, Phenyl-$(C_1-C_4)$-alkyl, wobei der Phenylring unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(3) Wasserstoff, $(C_1-C_{15})$-Alkyl, geradkettig oder verzweigt, $(C_3-C_{15})$-Alkenyl, geradkettig oder verzweigt, $(C_4-C_8)$-Cycloalkyl, $(C_4-C_8)$-Cycloalkyl-$(C_1-C_4)$alkyl, Phenyl oder Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(4) und R(4)', gleich oder verschieden und voneinander unabhängig, Wasserstoff, $(C_1-C_4)$-Alkyl, $(C_1-C_3)$-Alkoxy, F, Cl, $CF_3$, Nitro, Hydroxy, Acetamido oder Amino,

A die Gruppe $-(CH_2)_m-O-(CH_2)_n-$, worin m und n jeweils 2 oder 3 ist, oder eine der Gruppen $-(CH_2)_p-CH(OH)-(CH_2)_q-$ und $-(CH_2)_p-CH(OCH_3)-(CH_2)_q$, worin p und q jeweils 1, 2 oder 3 ist, oder

die Gruppe $-(CH_2)_r-O-CH_2-CO-$, worin r 2 oder 3 ist, oder die Gruppe $-(CH_2)_s-CO-$, worin s 1, 2, 3 oder 4 ist,

R(5) eine der folgenden Gruppen

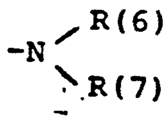 , 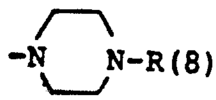 , 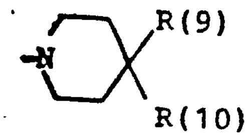 ,

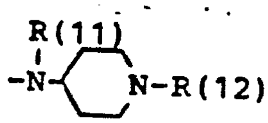 oder 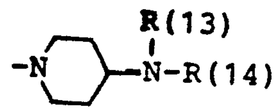 ,

worin

R(6) und R(7), gleich oder verschieden, voneinander unabhängig, Wasserstoff, $(C_1-C_{10})$-Alkyl, $(C_4-C_8)$-Cycloalkyl, $(C_4-C_8)$-Cycloalkyl-$(C_1-C_4)$-alkyl, Pyridyl-$(C_1-C_4)$-alkyl, Phenyl-$(C_1-C_6)$-alkyl, Benzhydryl oder Benzhydryl-$(C_1-C_4)$-alkyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(8) Wasserstoff, $(C_1-C_{10})$-Alkyl, geradkettig oder verzweigt, $(C_1-C_8)$-Alkanoyl, Pyridyl, Pyrimidinyl, Phenyl, Phenyl-$(C_1-C_4)$-alkyl, Phenyl-$(C_3-C_5)$-alkenyl, Benzhydryl oder Benzylhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(9) Wasserstoff, $(C_1-C_{10})$-Alkyl, Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$ Alkoxy, $(C_1-C_2)$-Alkylendioxy,

F, Cl, Br, $CF_3$ oder Hydroxy substituiert ist,

R(10) Wasserstoff, Hydroxy, oder $(C_1-C_4)$-Alkoxy, und

R(11) und R(12) bzw. R(13) und R(14), gleich oder verschieden und voneinander unabhängig, Wasserstoff, $(C_1-C_{10})$-Alkyl, geradkettig oder verzweigt, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl, oder Benzoyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind.

sowie die Salze der Verbindungen der Formel I mit pharmazeutisch akzeptablen Säuren.

2. Verbindung der Formel I nach Anspruch 1, in welcher

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido,

R(1)" Wasserstoff,

R(2) Wasserstoff, $(C_1-C_6)$-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, $(C_1-C_{12})$-Alkyl, geradkettig oder verzweigt, Allyl, Methallyl, $(C_5-C_7)$Cycloalkyl, $(C_5-C_7)$-Cycloalkyl-$(C_1-C_4)$-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor, Nitro, Hydroxy, Acetamido oder Amino,

R(4)' Wasserstoff,

A die Gruppe $-(CH_2)_m-O-(CH_2)_n-$, worin m und n jeweils 2 oder 3 ist, oder eine der Gruppen $-(CH_2)_p-CH(OH)-(CH_2)_q-$ und $-(CH_2)_p-CH(OCH_3)-(CH_2)_q$ worin p und q jeweils 1, 2 oder 3 ist, oder die Gruppe $-(CH_2)_r-O-CH_2-CO-$, worin r 2 oder 3 sein

kann, oder die Gruppe $-(CH_2)_s-CO-$, worin s 1, 2, 3 oder 4 ist,

R(5) eine der folgenden Gruppen

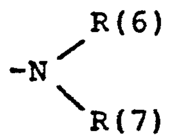
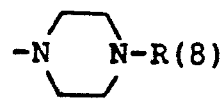
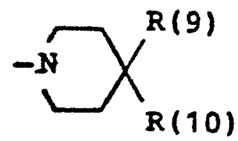

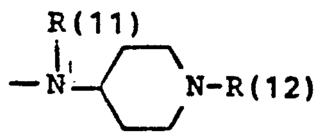 oder 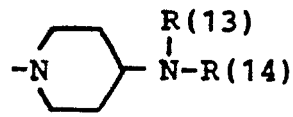

worin

R(6) Wasserstoff, Methyl, Ethyl, Propyl, Isopropyl,

R(7) Wasserstoff, Methyl, Ethyl, Propyl, Isopropyl, Cyclopentylethyl, Cyclohexylethyl, Phenyl-$(C_1-C_4)$-alkyl, Benzhydryl oder Benzhydryl-$(C_1-C_4)$-alkyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert sind, Pyridyl-$(C_1-C_4)$-alkyl,

R(8) Wasserstoff, $(C_1-C_6)$-Alkyl, geradkettig oder verzweigt, $(C_1-C_6)$-Alkanoyl, Phenyl, wobei der Phenylrest durch einen oder zwei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert sein kann, Phenyl-$(C_1-C_4)$-alkyl, Phenyl-$(C_3-C_5)$-alkenyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Ethyl, Methoxy, Ethoxy, $(C_1-C_2)$-Alkylendioxy, F, Cl, $CF_3$ oder Hydroxy substituiert sind,

R(9) Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist,

R(10) Wasserstoff, Hydroxy oder Methoxy,

R(11), R(12), R(13) und R(14) gleich oder verschieden und Wasserstoff, $(C_1-C_8)$-Alkyl, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste der Gruppe Methyl, Ethyl, Methoxy, Ethoxy, $(C_1-C_2)$-Alkylendioxy, F, Cl, $CF_3$ oder Hydroxy substituiert sind.

3. Verbindung der Formel I nach Anspruch 1, in welcher

R(1) Wasserstoff, Methyl, Methoxy, Fluor oder Chlor,

R(1)' Wasserstoff oder Methoxy,

R(1)" Wasserstoff,

R(2) Wasserstoff, Methyl, Ethyl, Propyl, Isopropyl, Butyl, sec. Butyl, Isobutyl, Benzyl, Phenethyl,

R(3) Wasserstoff $(C_1-C_{12})$-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl,

R(4) Wasserstoff, Methoxy, Methyl, Chlor, Nitro oder Hydroxy,

R(4)' Wasserstoff,

A die Gruppe $-(CH_2)_m-O-(CH_2)_n-$, worin m und n jeweils 2 oder 3 ist, oder eine der Gruppen $-(CH_2)_p-CH(OH)-(CH_2)_q-$ und $-(CH_2)_p-CH(OCH_3)-(CH_2)_q$ worin p 1 oder 2 und q 1 ist, oder die Gruppe $-(CH_2)_r-O-CH_2-CO-$, worin r 2 oder 3 ist, oder die Gruppe $-(CH_2)_s-CO-$, worin s 3 oder 4 ist,

R(5) eine der folgenden Gruppen

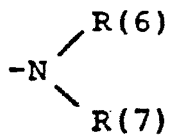 , 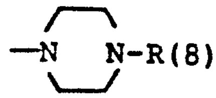 , 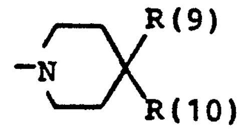 ,

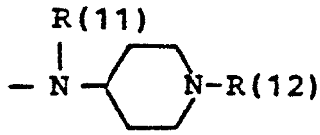 oder 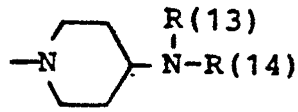 ,

worin bedeuten

R(6) Wasserstoff oder Methyl

R(7) Phenyl-($C_1$-$C_4$)-alkyl, Benzhydryl oder Benzhydryl-($C_1$-$C_4$)-alkyl, wobei der Phenylrest jeweils unsubstituiert oder durch einen, zwei oder drei Reste aus der Gruppe Methyl, Methoxy, Fluor, Chlor, Methylendioxy oder Hydroxy substituiert ist

R(8) ($C_1$-$C_6$)-Alkyl, geradkettig oder verzweigt, ($C_1$-$C_6$)-Alkanoyl, Phenyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Methoxy, Ethoxy, Methylendioxy, Fluor, Chlor oder Hydroxy substituiert sind,

R(9) Phenyl, wobei der Phenylrest unsubstituiert oder durch einen, zwei oder drei Reste aus der Gruppe Methyl, Methoxy, Fluor, Chlor, Methylendioxy oder Hydroxy substituiert ist,

R(10) Wasserstoff, Hydroxy oder Methoxy,

R(11), R(12), R(13) und R(14) gleich oder verschieden sind und Wasserstoff, ($C_1$-$C_6$)-Alkyl, ($C_1$-$C_6$)-Alkanoyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Methoxy, Methylendioxy, Fluor, Chlor oder Hydroxy substituiert sind.

4. Verfahren zum Herstellen einer Verbindung I nach Anspruch 1, dadurch gekennzeichnet, daß man

a) eine Verbindung der Formel II,

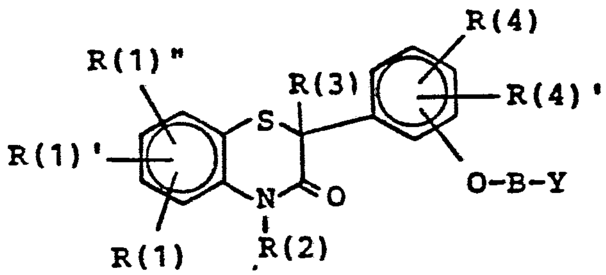

(II) ,

in welcher R(1), R(1)', R(1)", R(2), R(3), R(4) und R(4)' die gleiche Bedeutung wie in Formel I haben und in welcher B die Reste $-(CH_2)_m-O-(CH_2)_n$ oder $-(CH_2)_p-CH(OH)-(CH_2)_q$ bedeutet, wobei m, n, p und q die gleiche Bedeutung wie in Formel I haben, und in welcher Y eine Abgangsgruppe, die nucleophil verdrängt werden kann, bedeutet, mit einer der Verbindungen der Formeln IIIa, IIIb, IIIc, IIId oder IIIe

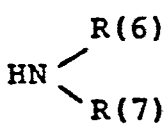

IIIa

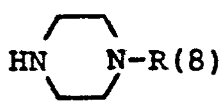

IIIb

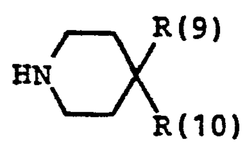

IIIc

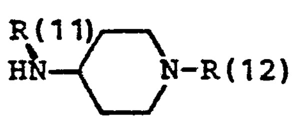

IIId

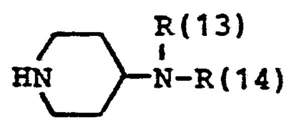

IIIe

in welchen R(6), R(7), R(8), R(9), R(10), R(11), R(12), R(13) und R(14) die gleiche Bedeutung wie in Formel I haben, unter Bedingungen einer nucleophilen Substitution, vorzugsweise in einem polaren organischen Lösungsmittel oder einem Kohlenwasserstoff mit oder ohne Gegenwart einer Hilfsbase zum Abfangen der sich bildenden Säure, bei einer Temperatur zwischen 0 und 160°C, umsetzt, oder daß man

0243886
HOE 86/F 087

b) eine Verbindung der Formel IV,

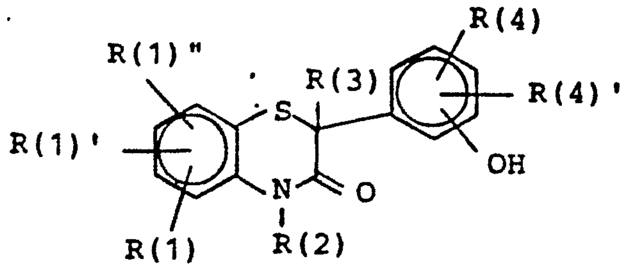

IV

in welcher R(1), R(1)', R(1)", R(2), R(3), R(4) und R(4)' die gleiche Bedeutung wie in Formel I haben, mit einer Verbindung der Formel V,

$$Z - A - R(5) \qquad V$$

in welcher Z gleich wie Y in Formel II definiert ist und in welcher R(5), und A die gleiche Bedeutung wie in Formel I haben, entweder in einem polaren aprotischen Lösungsmittel in Gegenwart einer starken Base bei einer Temperatur zwischen -40 und +60°C oder in einem protischen oder aprotischen polaren organischen Lösungsmittel in Gegenwart einer schwachen bis mittelstarken Base oder einem Amin bei einer Temperatur zwischen 0 und 160°C umsetzt, oder daß man

c) eine Verbindung der Formel VI

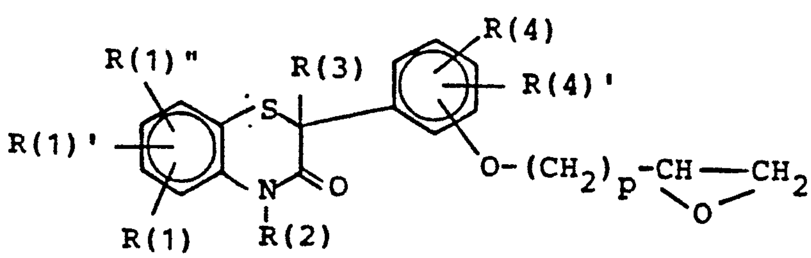

in welcher R(1), R(1)', R(1)" , R(2), R(3), R(4), R(4)' und p die gleiche Bedeutung wie in Formel I haben, mit Aminen der Formel IIIa - IIIe ohne Lösungsmittel oder in Gegenwart eines vorzugsweise polaren Lösungsmitttels umsetzt, wobei Verbindungen der Formel I entstehen, worin
A = $(CH_2)_p-CH(OH)-CH_2$ bedeutet, oder daß man

d) eine Verbindung der Formel VII

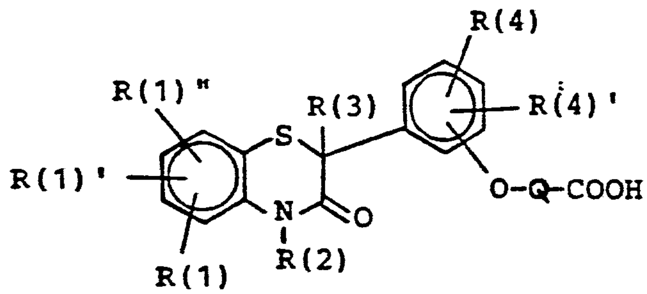

VII

in welcher R(1), R(1)', R(1)", R(2), R(3), R(4), R(4)' die gleiche Bedeutung wie in Formel I haben und in welcher Q die Reste $-(CH_2)_r-O-CH_2-CO-$ oder $-(CH_2)_s-CO-$, bedeutet wobei r und s die gleiche Bedeutung wie in Formel I haben,
mit einer der Verbindungen der Formeln IIIa, IIIb, IIIc, IIId oder IIIe unter üblichen Amidbildungsbedingungen umsetzt, wobei Verbindungen der Formel I entstehen, in denen A $-(CH_2)_r-O-CH_2CO-$ oder $-(CH_2)_s-CO-$ bedeutet.

5. Arzneimittel, dadurch gekennzeichnet, daß es eine Verbindung nach Anspruch 1 enthält oder daraus besteht.

6. Verwendung einer Verbindung I nach Anspruch 1 zur Behandlung von Störungen im Calciumhaushalt eines menschlichen oder tierischen Organismus.

Patentansprüche Griechenland, Österreich und Spanien:

1. Verfahren zum Herstellen einer Verbindung I sowie von deren Salzen mit pharmazeutisch akzeptablen Säuren

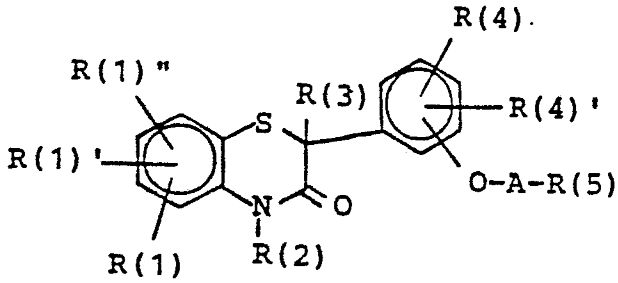

(I),

worin bedeuten

R(1), R(1)' und R(1)", gleich oder verschieden und voneinander unabhängig, Wasserstoff, $(C_1-C_4)$-Alkyl, $(C_1-C_3)$-Alkoxy, F, Cl, Br, $CF_3$, Nitro, Hydroxy, Acetamido oder Amino,

R(2) Wasserstoff, $(C_1-C_{10})$-Alkyl, geradkettig oder verzweigt, $(C_3-C_{10})$-Alkenyl, geradkettig oder verzweigt, Phenyl-$(C_1-C_4)$-alkyl, wobei der Phenylring unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(3) Wasserstoff, $(C_1-C_{15})$-Alkyl, geradkettig oder verzweigt, $(C_3-C_{15})$-Alkenyl, geradkettig oder verzweigt, $(C_4-C_8)$-Cycloalkyl, $(C_4-C_8)$-Cycloalkyl-$(C_1-C_4)$alkyl, Phenyl oder Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(4) und R(4)', gleich oder verschieden und voneinander unabhängig, Wasserstoff, $(C_1-C_4)$-Alkyl, $(C_1-C_3)$-Alkoxy, F, Cl, $CF_3$, Nitro, Hydroxy, Acetamido oder Amino,

A die Gruppe $-(CH_2)_m-O-(CH_2)_n-$, worin m und n jeweils 2 oder 3 ist, oder eine der Gruppen $-(CH_2)_p-CH(OH)-(CH_2)_q-$ und $-(CH_2)_p-CH(OCH_3)-(CH_2)_q$ worin p und q jeweils 1, 2 oder 3 ist, oder die Gruppe $-(CH_2)_r-O-CH_2-CO-$, worin r 2 oder 3 ist,

oder die Gruppe $-(CH_2)_s-CO-$, worin s 1, 2, 3 oder 4 ist,

R(5) eine der folgenden Gruppen

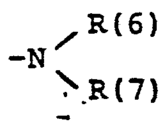 , 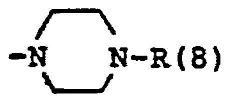 , 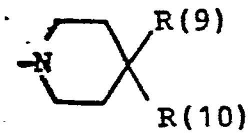 ,

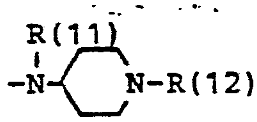 oder 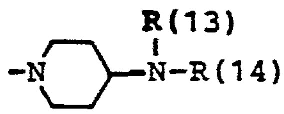 ,

worin

R(6) und R(7), gleich oder verschieden, voneinander unabhängig, Wasserstoff, $(C_1-C_{10})$-Alkyl, $(C_4-C_8)$-Cycloalkyl, $(C_4-C_8)$-Cycloalkyl-$(C_1-C_4)$-alkyl, Pyridyl-$(C_1-C_4)$-alkyl, Phenyl-$(C_1-C_6)$-alkyl, Benzhydryl oder Benzhydryl-$(C_1-C_4)$-alkyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(8) Wasserstoff, $(C_1-C_{10})$-Alkyl, geradkettig oder verzweigt, $(C_1-C_8)$-Alkanoyl, Pyridyl, Pyrimidinyl, Phenyl, Phenyl-$(C_1-C_4)$-alkyl, Phenyl-$(C_3-C_5)$-alkenyl, Benzhydryl oder Benzylhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(9) Wasserstoff, $(C_1-C_{10})$-Alkyl, Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$ Alkoxy, $(C_1-C_2)$-Alkylendioxy, F, Cl, Br, $CF_3$ oder Hydroxy substituiert ist,

R(10) Wasserstoff, Hydroxy, oder $(C_1-C_4)$-Alkoxy, und

R(11) und R(12) bzw. R(13) und R(14), gleich oder verschieden und voneinander unabhängig, Wasserstoff, $(C_1-C_{10})$-Alkyl, geradkettig oder verzweigt, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl, oder Benzoyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

dadurch gekennzeichnet, daß man

a) eine Verbindung der Formel II

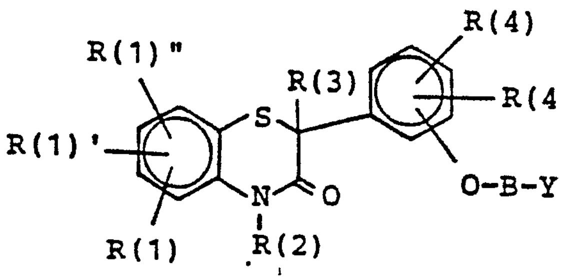

(II),

in welcher R(1), R(1)', R(1)", R(2), R(3), R(4) und R(4)' die gleiche Bedeutung wie in Formel I haben und in welcher B die Reste $-(CH_2)_m-O-(CH_2)_n$ oder $-(CH_2)_p-CH(OH)-(CH_2)_q$ bedeutet, wobei m, n, p und q die gleiche Bedeutung wie in Formel I haben, und in welcher Y eine Abgangsgruppe, die nucleophil verdrängt werden kann, bedeutet, mit einer der Verbindungen der Formeln IIIa, IIIb, IIIc, IIId oder IIIe

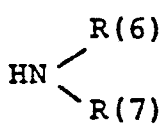

IIIa

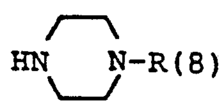

IIIb

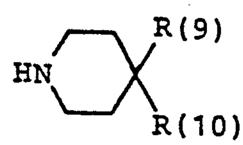

IIIc

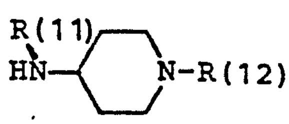

IIId

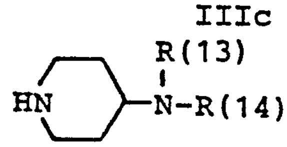

IIIe

in welchen R(6), R(7), R(8), R(9), R(10), R(11), R(12), R(13) und R(14) die gleiche Bedeutung wie in Formel I haben, unter

Bedingungen einer nucleophilen Substitution, vorzugsweise in einem polaren organischen Lösungsmittel oder einem Kohlenwasserstoff mit oder ohne Gegenwart einer Hilfsbase zum Abfangen der sich bildenden Säure, bei einer Temperatur zwischen 0 und 160°C, umsetzt, oder daß man

b) eine Verbindung der Formel IV,

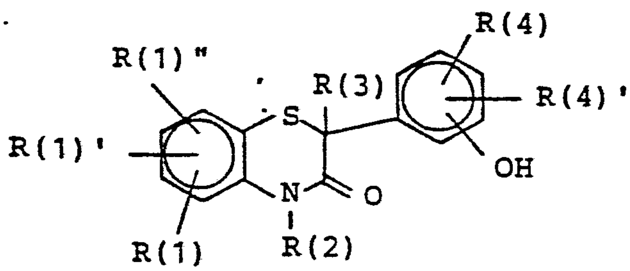

in welcher R(1), R(1)', R(1)", R(2), R(3), R(4) und R(4)' die gleiche Bedeutung wie in Formel I haben, mit einer Verbindung der Formel V,

Z - A - R(5) V

in welcher Z gleich wie Y in Formel II definiert ist und in welcher R(5), und A die gleiche Bedeutung wie in Formel I haben, entweder in einem polaren aprotischen Lösungsmittel in Gegenwart einer starken Base bei einer Temperatur zwischen -40 und +60°C oder in einem protischen oder aprotischen polaren organischen Lösungsmittel in Gegenwart einer schwachen bis mittelstarken Base oder einem Amin bei einer Temperatur zwischen 0 und 160°C umsetzt, oder daß man

c) eine Verbindung der Formel VI

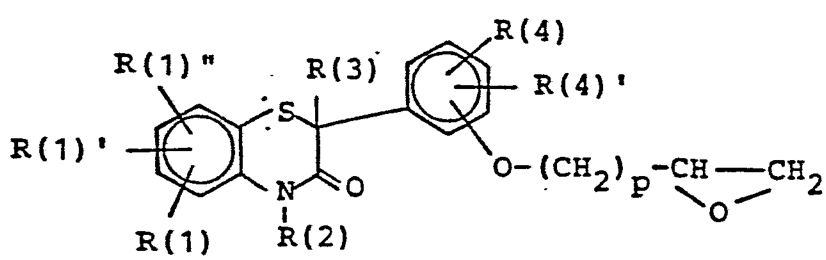

in welcher R(1), R(1)', R(1)", R(2), R(3), R(4), R(4)' und p die gleiche Bedeutung wie in Formel I haben, mit Aminen der Formel IIIa - IIIe ohne Lösungsmittel oder in Gegenwart eines vorzugsweise polaren Lösungsmitttels umsetzt, wobei Verbindungen der Formel I entstehen, worin $A = (CH_2)_p-CH(OH)-CH_2$ bedeutet, oder daß man

d) eine Verbindung der Formel VII

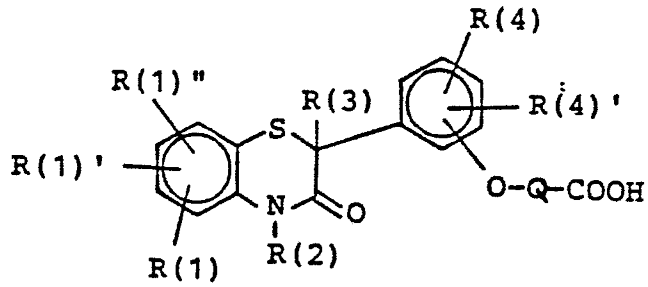

VII,

in welcher R(1), R(1)', R(1)", R(2), R(3), R(4), R(4)' die gleiche Bedeutung wie in Formel I haben und in welcher Q die Reste $-(CH_2)_r-O-CH_2-CO-$ oder $-(CH_2)_s-CO-$, bedeutet wobei r und s die gleiche Bedeutung wie in Formel I haben,
mit einer der Verbindungen der Formeln IIIa, IIIb, IIIc, IIId oder IIIe unter üblichen Amidbildungsbedingungen umsetzt, wobei Verbindungen der Formel I entstehen, in denen A $-(CH_2)_r-O-CH_2CO-$ oder $-(CH_2)_s-CO-$ bedeutet.

2. Verfahren nach Anspruch 1, bei welchem bedeuten:

R(1) und R(1)', gleich oder verschieden und voneinander unabhängig, Wasserstoff, Methyl, Ethyl, Methoxy, Ethoxy, Fluor, Chlor, $CF_3$, Nitro oder Acetamido,

R(1)" Wasserstoff,

R(2) Wasserstoff, $(C_1-C_6)$-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, $(C_1-C_{12})$-Alkyl, geradkettig oder verzweigt, Allyl, Methallyl, $(C_5-C_7)$Cycloalkyl, $(C_5-C_7)$-

Cycloalkyl-($C_1$-$C_4$)-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor, Nitro, Hydroxy, Acetamido oder Amino,

R(4)' Wasserstoff,

A die Gruppe $-(CH_2)_m-O-(CH_2)_n-$, worin m und n jeweils 2 oder 3 ist, oder eine der Gruppen $-(CH_2)_p-CH(OH)-(CH_2)_q-$ und $-(CH_2)_p-CH(OCH_3)-(CH_2)_q$ worin p und q jeweils 1, 2 oder 3 ist, oder die Gruppe $-(CH_2)_r-O-CH_2-CO-$, worin r 2 oder 3 sein kann, oder die Gruppe $-(CH_2)_s-CO-$, worin s 1, 2, 3 oder 4 ist

R(5) eine der folgenden Gruppen

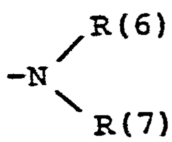 ; 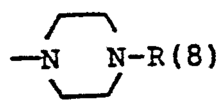 , 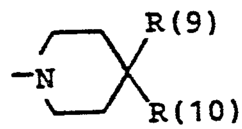 ,

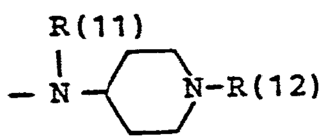 oder 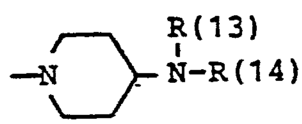 ,

worin

R(6) Wasserstoff, Methyl, Ethyl, Propyl, Isopropyl,

R(7) Wasserstoff, Methyl, Ethyl, Propyl, Isopropyl, Cyclopentylethyl, Cyclohexylethyl, Phenyl-($C_1$-$C_4$)-alkyl, Benzhydryl oder Benzhydryl-($C_1$-$C_4$)-alkyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert sind, Pyridyl-($C_1$-$C_4$)-alkyl,

R(8) Wasserstoff, ($C_1$-$C_6$)-Alkyl, geradkettig oder verzweigt, ($C_1$-$C_6$)-Alkanoyl, Phenyl, wobei der Phenylrest durch einen oder zwei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert sein kann, Phenyl-($C_1$-$C_4$)-alkyl, Phenyl-($C_3$-$C_5$)-alkenyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Ethyl, Methoxy, Ethoxy, $(C_1-C_2)$-Alkylendioxy, F, Cl, $CF_3$ oder Hydroxy substituiert sind,

R(9) Phenyl, Phenyl-$(C_1-C_4)$-alkyl, wobei der Phenylrest jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, $CF_3$ oder Hydroxy substituiert ist,

R(10) Wasserstoff, Hydroxy oder Methoxy,

R(11), R(12), R(13) und R(14) gleich oder verschieden und Wasserstoff, $(C_1-C_8)$-Alkyl, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste der Gruppe Methyl, Ethyl, Methoxy, Ethoxy, $(C_1-C_2)$-Alkylendioxy, F, Cl, $CF_3$ oder Hydroxy substituiert sind.

3. Verfahren nach Anspruch 1, bei welchem bedeuten:

R(1) Wasserstoff, Methyl, Methoxy, Fluor oder Chlor,

R(1)' Wasserstoff oder Methoxy,

R(1)" Wasserstoff,

R(2) Wasserstoff, Methyl, Ethyl, Propyl, Isopropyl, Butyl, sec. Butyl, Isobutyl, Benzyl, Phenethyl,

R(3) Wasserstoff $(C_1-C_{12})$-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl,

R(4) Wasserstoff, Methoxy, Methyl, Chlor, Nitro oder Hydroxy,

R(4)' Wasserstoff,

A die Gruppe $-(CH_2)_m-O-(CH_2)_n-$, worin m und n jeweils 2 oder 3 ist, oder eine der Gruppen $-(CH_2)_p-CH(OH)-(CH_2)_q-$ und $-(CH_2)_p-CH(OCH_3)-(CH_2)_q$ worin p 1 oder 2 und q 1 ist, oder die Gruppe $-(CH_2)_r-O-CH_2-CO-$, worin r 2 oder 3 ist, oder die Gruppe $-(CH_2)_s-CO-$, worin s 3 oder 4 ist,

R(5) eine der folgenden Gruppen

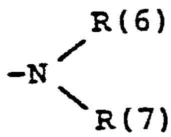 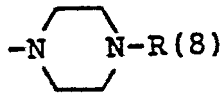 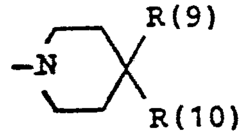

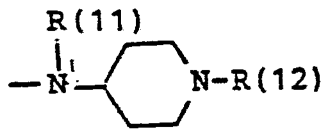 oder 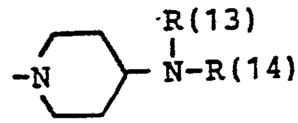

worin bedeuten

R(6) Wasserstoff oder Methyl

R(7) Phenyl-$(C_1-C_4)$-alkyl, Benzhydryl oder Benzhydryl-$(C_1-C_4)$-alkyl, wobei der Phenylrest jeweils unsubstituiert oder durch einen, zwei oder drei Reste aus der Gruppe Methyl, Methoxy, Fluor, Chlor, Methylendioxy oder Hydroxy substituiert ist

R(8) $(C_1-C_6)$-Alkyl, geradkettig oder verzweigt, $(C_1-C_6)$-Alkanoyl, Phenyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Methoxy, Ethoxy, Methylendioxy, Fluor, Chlor oder Hydroxy substituiert sind,

R(9) Phenyl, wobei der Phenylrest unsubstituiert oder durch einen, zwei oder drei Reste aus der Gruppe Methyl, Methoxy, Fluor, Chlor, Methylendioxy oder Hydroxy substituiert ist,

R(10) Wasserstoff, Hydroxy oder Methoxy,

R(11), R(12), R(13) und R(14) gleich oder verschieden sind und Wasserstoff, $(C_1-C_6)$-Alkyl, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Methoxy, Methylendioxy, Fluor, Chlor oder Hydroxy substituiert sind,
bedeuten.

4. Verfahren zum Herstellen eines Arzneimittels, dadurch gekennzeichnet, daß man eine nach Anspruch 1 erhaltene Verbindung mit pharmazeutisch üblichen Trägerstoffen mischt.

5. Verwendung einer nach Anspruch 1 erhaltenen Verbindung I zum Herstellen eines Medikaments zur Behandlung von Störungen im Calciumhaushalt eines menschlichen oder tierischen Organismus.